# EUROPEAN PATENT SPECIFICATION

(11) **EP 4 307 428 B1**
(45) Date of publication and mention of the grant of the patent: **28.01.2026**
(21) Application number: 23770954.8
(22) Date of filing: 30.01.2023
(51) Int. Cl.: H01M 10/0567, H01M 4/525, H01M 4/505, H01M 10/052, H01M 4/131, H01M 4/587, H01M 4/133, H01M 4/42, H01M 4/58, H01M 10/0525, H01M 10/42

(54) **NOVEL ADDITIVE FOR NON-AQUEOUS ELECTROLYTE SOLUTION AND LITHIUM SECONDARY BATTERY COMPRISING SAME**
NEUARTIGES ADDITIV FÜR NICHTWÄSSRIGE ELEKTROLYTLÖSUNG UND LITHIUMSEKUNDÄRBATTERIE DAMIT
NOUVEL ADDITIF POUR SOLUTION ÉLECTROLYTIQUE NON AQUEUSE ET BATTERIE SECONDAIRE AU LITHIUM LE COMPRENANT

(30) Priority: 17.03.2022 KR 20220033306
(43) Date of publication of application: 17.01.2024
(73) Proprietor: LG Energy Solution, Ltd., Seoul 07335 (KR)
(72) Inventor: JEONG, You Kyeong, Daejeon 34122 (KR); AHN, Kyoung Ho, Daejeon 34122 (KR); HAN, Jun Hyeok, Daejeon 34122 (KR); SHIN, Won Kyung, Daejeon 34122 (KR); LEE, Won Tae, Daejeon 34122 (KR); JI, Su Hyeon, Daejeon 34122 (KR); OH, Young Ho, Daejeon 34122 (KR)
(74) Representative: Hoffmann Eitle
(86) International application number: PCT/KR2023/001358
(87) International publication number: WO 2023/177074

(56) References cited:
- WO-A1-2018/059484
- JP-A- 2004 331 799
- KR-A- 20190 077 999
- US-A1- 2002 055 046
- RAHMAN K, ET AL.: "Synthesis, Characterization, and Copolymerization of a Series of Novel Acid Monomers Based on Sulfonimides for Proton Conducting Membranes", MACROMOLECULES, AMERICAN CHEMICAL SOCIETY, US, vol. 37, no. 15, 1 January 2004 (2004-01-01), US , pages 5575 - 5577, XP003018873, ISSN: 0024-9297, DOI: 10.1021/ma0498058
- FEDELI ELISABETTA, KVASHA ANDRIY, GIGMES DIDIER, PHAN TRANG N. T.: "Synthesis and Use of Zwitterion Bearing Sulfonyl(trifluoromethane sylfonyl)imide Anion as Additive for Polymer Electrolytes", APPLIED SCIENCES, vol. 10, no. 21, 31 October 2020 (2020-10-31), pages 7724, XP093091825, DOI: 10.3390/app10217724

## Description

### Technical Field

The present invention relates to an electrolyte composition and a lithium secondary battery including the same.

This application claims the benefit of priority based on Korean Patent Application No. 10-2022-0033306 filed on March 17, 2022.

### Background Technology of the Invention

In recent years, secondary batteries have been widely applied in small devices such as portable electronics, as well as in medium and large devices such as battery packs or power storage in hybrid and electric vehicles. These secondary batteries include lithium-ion batteries, lithium batteries, lithium-ion capacitors, and non-aqueous electrolytic liquid batteries such as sodium-ion batteries.

Among these non-aqueous electrolyte batteries, lithium-ion batteries are used by injecting an electrolyte solution into a battery cell including a positive electrode containing a positive electrode active material capable of intercalating and deintercalating lithium and a negative electrode containing a negative electrode active material capable of intercalating and deintercalating lithium. In particular, the electrolyte is an organic solvent in which lithium salts are dissolved and is important in determining the stability and performance of the lithium secondary battery.

For example, LiPF₆, which is the most commonly used lithium salt in electrolytes, reacts with the electrolyte solvent to generate HF while promoting the depletion of the solvent. This HF can not only generate a large amount of gas under high temperature conditions, but also elute metal ions, and if the eluted metal ions occur in the form of precipitation on the negative electrode surface, it will cause the negative electrode potential to rise and the cell OCV to fall, thereby reducing the performance of the battery as well as its lifetime and high temperature safety.

WO 2018/059484 A1 concerns a solid state electrolyte containing a polymer.

US 2017/058066 A1 concerns a conductive polymer and the production thereof.

### Technical Problem

Accordingly, the purpose of the present invention is to provide technology that can prevent direct contact between the positive electrode and the electrolyte by forming a film on the surface of the electrode, reduce the oxidative decomposition of the electrolyte by preventing direct contact between the positive electrode and HF, PF₅, etc. to suppress gas generation, improve the capacity retention rate by improving the phenomenon of metal ion precipitation from the positive electrode, and prevent the decline of the OCV of the battery.

### Technical Solution

To solve the above-mentioned problems, the present invention provides an electrolyte composition for a secondary battery including a non-aqueous organic solvent, a lithium salt, and a compound represented by Formula 1 below: 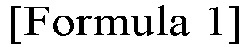 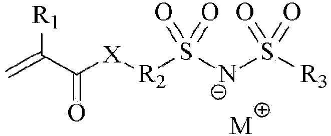
in Formula 1,
R₁ is hydrogen or an alkyl group having a carbon number of 1 to 4;
R₂ is an arylene group having from 6 to 20 carbons, an aryleneoxy group having from 6 to 20 carbons, a heteroarylene group having from 5 to 10 carbons containing at least one heteroatom among N, S, and O, or a heteroaryleneoxy group having from 5 to 10 carbons containing at least one heteroatom among N, S, and O;
R₃ is a fluoro group, an alkyl group having a carbon number of 1 to 10, an alkoxy group having a carbon number of 1 to 10, or 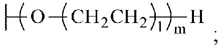 wherein at least one hydrogen included in the alkyl group having a carbon number of 1 to 10, alkoxy group having a carbon number of 1 to 10, and 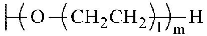 is optionally substituted by a fluorine atom;
X is an oxygen atom (O) or -NR₄;
R₄ is hydrogen or an alkyl group having a carbon number of 1 to 4;
M is at least one species selected from lithium, sodium, and potassium;
l is an integer from 1 to 6; and
m is an integer from 2 to 20.

Specifically, R₁ may be hydrogen or a methyl group,
R₂ may be a phenylene group, a naphthylene group, an anthracenyl group, a biphenylene group, a phenyleneoxy group, a pyridinylene group, a thiophenylene group, a dioxolene group, or a dithiolene group,
R₃ may be a fluoro group, a methyl group, an ethyl group, a propyl group, a methoxy group, an ethoxy group, 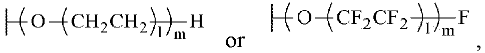
X may be an oxygen atom (O), -NH, or -NCH₃,
M may be lithium,
l may be an integer of 1 or 2, and
m may be an integer from 2 to 10.

More specifically, the compound represented by Formula 1 may be one or more compounds of <Structural Formula 1> to <Structural Formula 48> below:

| | | |
|---|---|---|
| Structural Formula 1 | Structural Formula 2 | Structural Formula 3 |
| 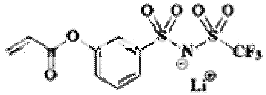 | 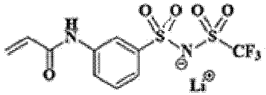 | 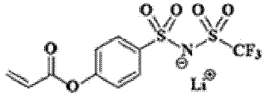 |
| Structural Formula 4 | Structural Formula 5 | Structural Formula 6 |
| 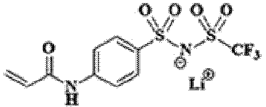 | 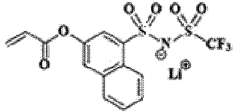 | 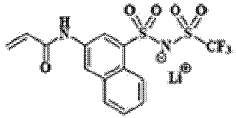 |
| Structural Formula 7 | Structural Formula 8 | Structural Formula 9 |
| 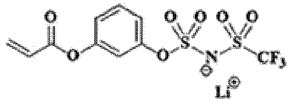 | 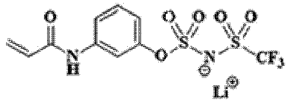 | 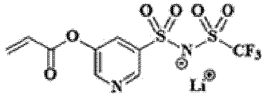 |
| Structural Formula 10 | Structural Formula 11 | Structural Formula 12 |
| 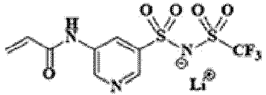 | 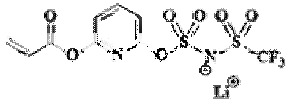 | 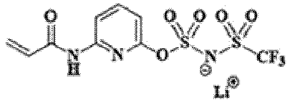 |
| Structural Formula 13 | Structural Formula 14 | Structural Formula 15 |
| 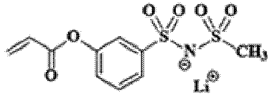 | 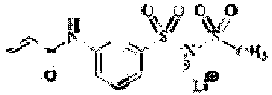 | 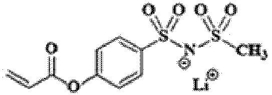 |
| Structural Formula 16 | Structural Formula 17 | Structural Formula 18 |
| 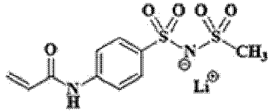 | 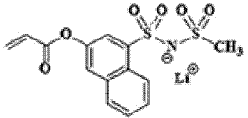 | 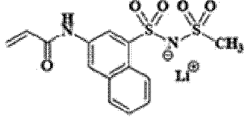 |
| Structural Formula 19 | Structural Formula 20 | Structural Formula 21 |
| 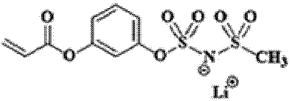 | 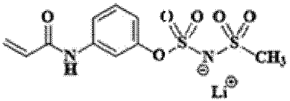 | 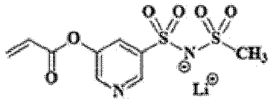 |
| Structural Formula 22 | Structural Formula 23 | Structural Formula 24 |
| 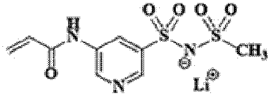 | 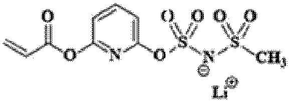 | 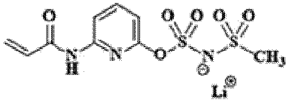 |
| Structural Formula 25 | Structural Formula 26 | Structural Formula 27 |
| 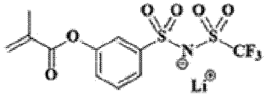 | 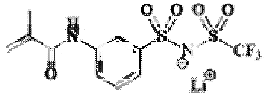 | 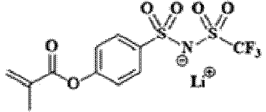 |
| Structural Formula 28 | Structural Formula 29 | Structural Formula 30 |
| 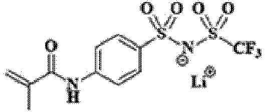 | 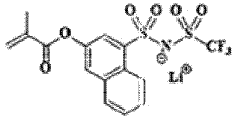 | 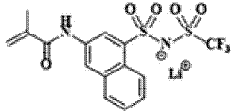 |
| Structural Formula 31 | Structural Formula 32 | Structural Formula 33 |
| 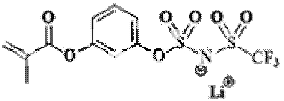 | 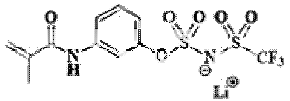 | 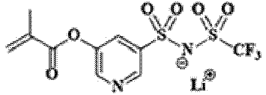 |
| Structural Formula 34 | Structural Formula 35 | Structural Formula 36 |
| 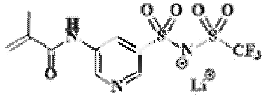 | 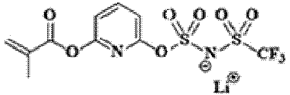 | 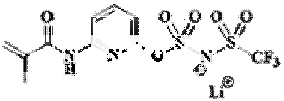 |
| Structural Formula 37 | Structural Formula 38 | Structural Formula 39 |
| 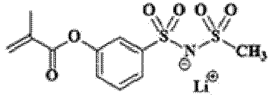 | 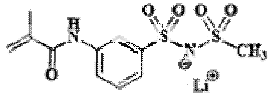 | 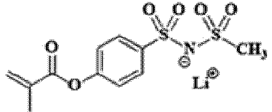 |
| Structural Formula 40 | Structural Formula 41 | Structural Formula 42 |
| 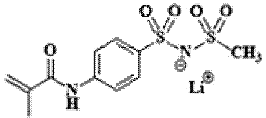 | 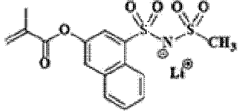 | 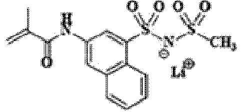 |
| Structural Formula 43 | Structural Formula 44 | Structural Formula 45 |
| 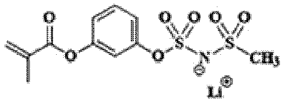 | 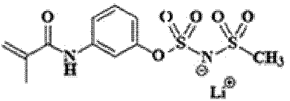 | 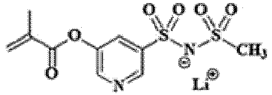 |
| Structural Formula **46** | Structural Formula 47 | Structural Formula 48 |
| 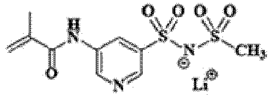 | 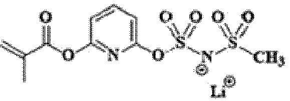 | 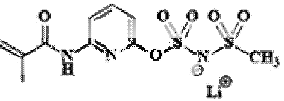 |

The compound represented by Formula 1 may be included in an amount of 0.01 to 3 wt% based on the total weight of the electrolyte composition.

In addition, the lithium salt may include at least one species of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

Moreover, the non-aqueous organic solvent may include at least one species of N-methyl-2-pyrrolidinone, ethylene carbonate, propylene carbonate, butylene carbonate, dimethyl carbonate, diethyl carbonate, gamma-butyrolactone, 1,2-dimethoxyethane, tetrahydrofuran, 2-methyl tetrahydrofuran, dimethyl sulfoxide, 1,3-dioxolane, formamide, and dimethylformamide, dioxolane, acetonitrile, nitromethane, methyl formate, methyl acetate, phosphoric acid triester, trimethoxymethane, dioxolane derivative, sulfurane, methyl sulfurane, 1,3-dimethyl-2-imidazolidinone, propylene carbonate derivative, tetrahydrofuran derivative, ether, methyl pyrophosphate, and ethyl propionate.

Furthermore, the present invention provides a lithium secondary battery comprising:
an electrode assembly comprising a positive electrode comprising at least one among lithium metal oxides represented by Formula 2 and Formula 3 below, a negative electrode comprising negative electrode active material, and a separator interposed between the positive electrode and the negative electrode; and
the electrolyte composition according to the present invention:

   [Formula 2] Lix[Ni_{y}Co_{z}Mn_{w}M¹ᵥ]O₂

   [Formula 3] LiM²ₚMn₍₂₋ₚ₎O₄
in Formula 2 and Formula 3,
M¹ is at least one element selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w, and v are respectively 1.0≤x≤1.30, 0.5≤y<1, 0<z≤0.3, 0<w≤0.3, 0≤v≤0.1, y+z+w+v=1,
M² is Ni, Co, or Fe, and
p is 0.05≤p≤0.6.

Here, the positive electrode active material may comprise at least one species selected from LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}CO_{0.2}Mn_{0.15}Al_{0.05}O₂, LiNi_{0.7}Co_{0.1}Mn_{0.1}Al_{0.1}O₂, and LiNi_{0.3}Mn_{1.5}O₄.

In addition, the negative electrode active material may comprise a carbon material and a silicon material, wherein the silicon material includes at least one of silicon (Si), silicon carbide (SiC), and silicon oxide (SiO_{q}, wherein 0.8≤q≤2.5).

Moreover, the silicon material may be included at 1 to 20 wt% based on the total weight of the negative electrode active material.

### Advantageous Effects

The electrolyte additive according to the present invention can prevent a large amount of gas from being generated under high temperature conditions by forming a film on the surface of the electrode during the activation of the secondary battery, and can effectively prevent metal ions from eluting from the electrode, which results in a decrease in the OCV of the cell and a decrease in the capacity retention rate, thereby effectively improving the durability, performance and high temperature safety of the cell.

### Best Mode for Carrying Out the invention

The present invention is subject to various modifications and can have many embodiments, and specific embodiments are described in detail in the following description.

However, this is not intended to limit the invention to any particular embodiment and should be understood to include all modifications, equivalents and variations within the scope of the invention, as defined by the claims.

In the present invention, the terms "includes" or "has" are intended to designate the presence of the features, numbers, steps, actions, components, parts, or combinations thereof recited in the specification, and not to preclude the presence or absence of one or more other features, numbers, steps, actions, components, parts, or combinations thereof.

Also, in the present invention, when a part of a layer, membrane, area, plate, etc. is described as being "above" another part, this includes not only when it is "directly above" another part, but also when there is another part in between. Conversely, when a part of a layer, membrane, area, plate, etc. is described as being "below" another part, this includes not only when it is "directly below" another part, but also when there is another part in between. Also, in the present application, being disposed "on top" may include not only being disposed on top, but also being disposed on the bottom.

Also, in the present invention, "comprising as a major component" may mean comprising at least 50 wt%, at least 60 wt%, at least 70 wt%, at least 80 wt%, at least 90 wt%, or at least 95 wt% of a defined component relative to the total weight. For example, "comprising graphite as the primary component of the negative electrode active material" may mean comprising at least 50 wt%, at least 60 wt%, at least 70 wt%, at least 80 wt%, at least 90 wt%, or at least 95 wt% graphite based on the total weight of the negative electrode active material, and in some cases may mean that the entire negative electrode active material is composed of graphite and comprises 100 wt% graphite.

Hereinafter, the present invention will be described in more detail.

### Electrolyte additive

The compound of Formula 1 used as an additive in the electrolyte composition for a secondary battery according to the present invention includes a sulfonylimide group on one side as shown in Formula 1 above, and is an ionic compound with parent nucleus structure combined by a cyclic unsaturated hydrocarbon group having a (meth)acrylate group or (meth)acrylamide group having a conjugated structure, or a cyclic unsaturated hydrocarbon group having a conjugated structure, or a cyclic unsaturated heterocyclic hydrocarbon group having a structure in which a heteroatom is introduced into the cyclic unsaturated hydrocarbon group. The compound may include, as a linker between the sulfonylimide group and the (meth)acrylate or (meth)acrylamide group, a cyclic unsaturated hydrocarbon group having a conjugated structure or a functional group of a structure in which a heteroatom is introduced into said cyclic unsaturated hydrocarbon group, so that a conjugated structure can be implemented from the sulfonylimide group to the (meth)acrylate or (meth)acrylamide group, so when a secondary battery comprising the same is activated, an organic and/or inorganic film can be uniformly formed on the surface of the positive electrode and/or the negative electrode. In other words, the electrolyte additive can be directly incorporated in monomolecular form into the organic and/or inorganic film formed on the surface of the positive electrode and/or the negative electrode upon activation of the secondary battery, thereby suppressing the decomposition of the electrolyte and the generation of gas when the battery is exposed to high temperature, and improving the battery OCV and capacity retention rate drop phenomenon generated at the positive electrode.

For this purpose, in the compound represented by Formula 1 above,
R₁ may be hydrogen, a methyl group, an ethyl group or a propyl group,
R₂ may be a phenylene group, a naphthylene group, an anthracenyl group, a biphenylene group, a phenyleneoxy group, a pyridinylene group, a thiophenylene group, a dioxolene group, or a dithiolene group,
R₃ may be a fluoro group, a methyl group, an ethyl group, a propyl group, a methoxy group, an ethoxy group, 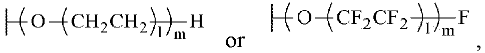 and
X may be an oxygen atom (O), -NH, -NCH₃, or -NCH₂CH₃.

Specifically, R₁ may be hydrogen or a methyl group,
R₂ may be a phenylene group, a naphthylene group, an anthracenyl group, a biphenylene group, a phenyleneoxy group, a pyridinylene group, a thiophenylene group, a dioxolene group, or a dithiolene group,
R₃ may be a fluoro group, a methyl group, an ethyl group, a propyl group, a methoxy group, an ethoxy group, 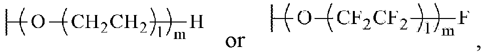
X may be an oxygen atom (O), -NH, or -NCH₃,
M may be lithium,
l may be an integer of 1 or 2, and
m may be an integer from 2 to 10.

As an example, the compound represented by Formula 1 may be one or more of the compounds of <Structural Formula 1> to <Structural Formula 48> below:

| | | |
|---|---|---|
| Structural Formula 1 | Structural Formula 2 | Structural Formula 3 |
| 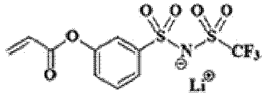 | 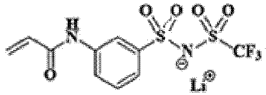 | 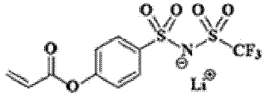 |
| Structural Formula 4 | Structural Formula 5 | Structural Formula 6 |
| 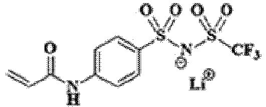 | 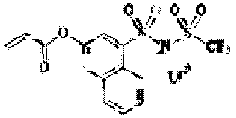 | 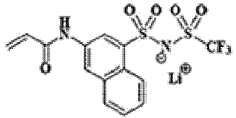 |
| Structural Formula 7 | Structural Formula 8 | Structural Formula 9 |
| 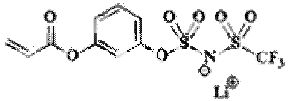 | 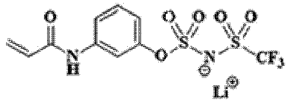 | 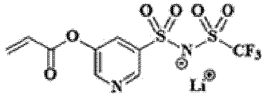 |
| Structural Formula 10 | Structural Formula 11 | Structural Formula 12 |
| 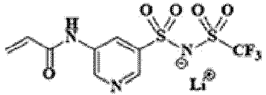 | 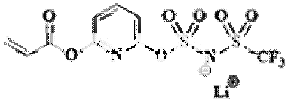 | 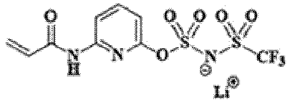 |
| Structural Formula 13 | Structural Formula 14 | Structural Formula 15 |
| 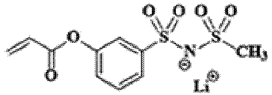 | 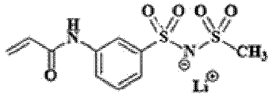 | 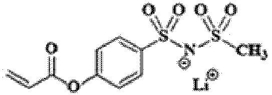 |
| Structural Formula 16 | Structural Formula 17 | Structural Formula 18 |
| 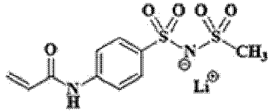 | 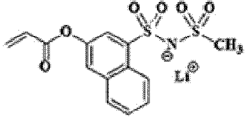 | 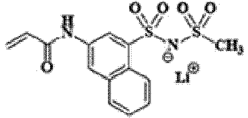 |
| Structural Formula 19 | Structural Formula 20 | Structural Formula 21 |
| 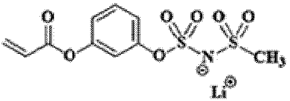 | 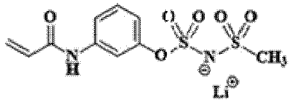 | 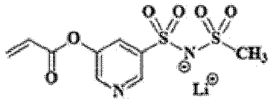 |
| Structural Formula 22 | Structural Formula 23 | Structural Formula 24 |
| 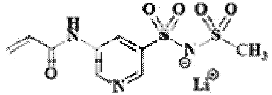 | 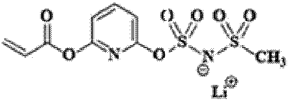 | 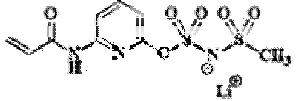 |
| Structural Formula 25 | Structural Formula 26 | Structural Formula 27 |
| 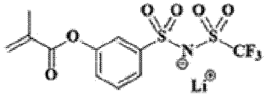 | 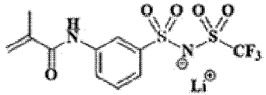 | 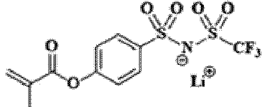 |
| Structural Formula 28 | Structural Formula 29 | Structural Formula 30 |
| 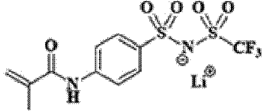 | 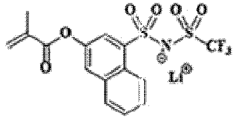 | 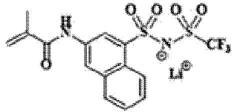 |
| Structural Formula 31 | Structural Formula 32 | Structural Formula 33 |
| 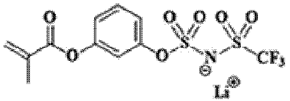 | 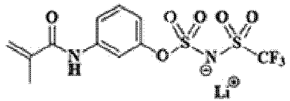 | 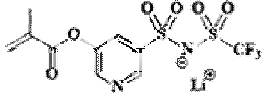 |
| Structural Formula 34 | Structural Formula 35 | Structural Formula 36 |
| 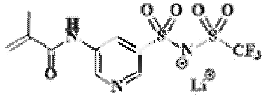 | 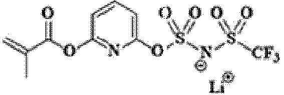 | 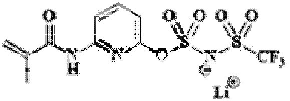 |
| Structural Formula 37 | Structural Formula 38 | Structural Formula 39 |
| 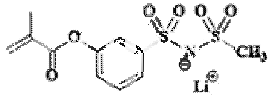 | 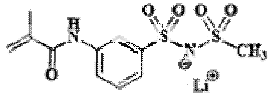 | 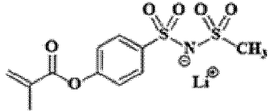 |
| Structural Formula 40 | Structural Formula 41 | Structural Formula 42 |
| 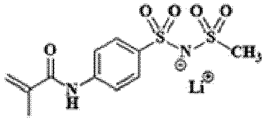 | 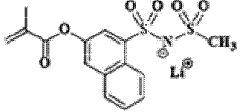 | 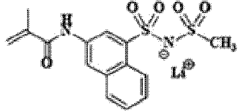 |
| Structural Formula 43 | Structural Formula 44 | Structural Formula 45 |
| 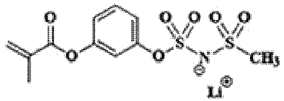 | 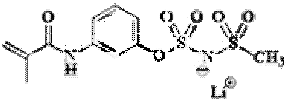 | 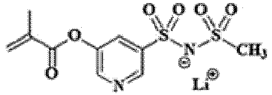 |
| Structural Formula 46 | Structural Formula 47 | Structural Formula 48 |
| 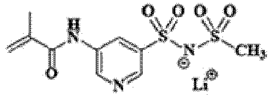 | 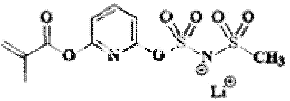 | 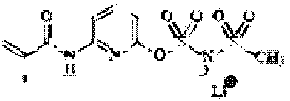 |

The electrolyte additive has a structure in which a sulfonylimide group, as described above, is on one side, and is bonded through a functional group with a structure in which a cyclic unsaturated hydrocarbon group having a (meth)acrylate group or (meth)acrylamide group having a conjugated structure, or a heteroatom is introduced into the cyclic unsaturated hydrocarbon group, so that there is an electric charge inside the molecule. The electrolyte additive with this structure can directly participate in the solvation shell of lithium ions even at low potentials during activation of the secondary battery, and can uniformly form a negatively charged and/or inorganic film through a reduction reaction on the negative electrode surface, while simultaneously forming an inorganic film through an oxidation reaction on the positive electrode surface. This organic and/or inorganic film can inhibit the decomposition of the electrolyte and the generation of gas when the cell is exposed to high temperatures, while improving the OCV decrease and capacity decrease of the cell generated at the positive electrode, thereby preventing the deterioration of the secondary battery.

### Electrolyte composition for lithium secondary battery

The electrolyte composition for a lithium secondary battery according to the present invention is a liquid, non-aqueous electrolyte composition having a composition including a lithium salt in a non-aqueous organic solvent and an electrolyte additive of Formula 1, as described above, which, upon activation of the battery including it, can directly participate in the solvation shell of lithium ions to uniformly form a negatively charged and/or inorganic film via a reduction reaction on the negative electrode surface.

The compound represented by Formula 1 may be included in the electrolyte composition at a specific content. Specifically, the compound represented by Formula 1 may be included at 0.01 to 5 wt% based on the total weight of the electrolyte composition, and more specifically, at 0.05 to 3 wt% or 1.0 to 2.5 wt% based on the total weight of the electrolyte composition. The present invention may utilize an excess of electrolyte additive wherein the content of the electrolyte additive is outside the above-described range to increase the viscosity of the electrolyte composition, thereby preventing a decrease in wettability to the electrodes and separator. Furthermore, it can prevent the battery performance from deteriorating by the decrease in ionic conductivity of the electrolyte composition, which is caused by an excessive amount of electrolyte additive inducing a polymerization through self-polymerization in the electrolyte composition when the battery is activated. In addition, the present invention may use a trace amount of electrolyte additive outside the range described above to prevent the effect of the additive from being implemented minimally.

Meanwhile, the lithium salt used in the electrolyte composition is not particularly limited to those used in the art for non-aqueous electrolytes. Specifically, the lithium salt may include one or more selected from LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, or (FSO₂)₂NLi.

As to the concentration of these lithium salts, there is no particular limitation, but a suitable concentration range has a lower limit of 0.5 mol/L or more, more particularly 0.7 mol/L or more, more particularly 0.9 mol/L or more, and an upper limit of 2.5 mol/L or less, more particularly 2.0 mol/L or less, more particularly 1.5 mol/L or less. When the concentration of the lithium salt is lower than 0.5 mol/L, the ionic conductivity is reduced, and the cycle characteristics and output characteristics of the non-aqueous electrolyte cell may be reduced. In addition, if the concentration of lithium salts exceeds 2.5 mol/L, the viscosity of the electrolyte for the non-aqueous electrolyte cell increases, which may also reduce the ionic conductivity and may reduce the cycle characteristics and output characteristics of the non-aqueous electrolyte cell.

In addition, when a large amount of lithium salt is dissolved in a non-aqueous organic solvent at one time, the liquid temperature may rise due to the heat of dissolution of the lithium salt. If the temperature of the non-aqueous organic solvent rises significantly due to the heat of dissolution of the lithium salts, there is a risk that fluorine-containing lithium salts may accelerate decomposition and produce hydrogen fluoride (HF). Therefore, the temperature at which the lithium salt is dissolved in the non-aqueous organic solvent is not particularly limited, but may be controlled from -20 to 80°C, and more particularly from 0 to 60°C.

Furthermore, the non-aqueous organic solvent used in the above electrolyte composition may be any non-aqueous organic solvent used in the art for non-aqueous electrolytes, without being particularly limited. Specifically, as the non-aqueous organic solvent, aprotic organic solvents such as N-methyl-2-pyrrolidinone, ethylene carbonate (EC), propylene carbonate, butylene carbonate, dimethyl carbonate (DMC), diethyl carbonate (DEC), gamma-butylolactone, 1,2-dimethoxyethane (DME), tetrahydrofuran, 2-methyl tetrahydrofuran, dimethyl sulfoxide, 1,3-dioxolane, and formamide, dimethylformamide, dioxolane, acetonitrile, nitromethane, methyl formate, methyl acetate, phosphoric acid triester, trimethoxymethane, dioxolane derivative, sulfurane, methyl sulfurane, 1,3-dimethyl-2-imidazolidinone, propylene carbonate derivative, tetrahydrofuran derivative, ether, methyl pyrophosphate, and ethyl propionate may be used.

In addition, the non-aqueous organic solvents utilized in the present invention may be one type alone, or two or more types may be mixed in any combination and proportion to suit the application. Among them, propylene carbonate, ethylene carbonate, fluoroethylene carbonate, diethyl carbonate, dimethyl carbonate, and ethyl methyl carbonate are particularly preferred from the viewpoint of electrochemical stability against redox and chemical stability against heat and reaction with solutes.

Meanwhile, the electrolyte composition may contain additives in addition to the basic components described above. Without harming to the essence of the present invention, additives commonly used in non-aqueous electrolytes of the present invention may be added in any proportion. Specifically, compounds having overcharge prevention effects, negative electrode film formation effects, and positive electrode protection effects such as cyclohexylbenzene, biphenyl, t-butylbenzene, carbonate, vinyl ethylene carbonate, difluoroanisole, fluoroethylene carbonate, propanesulfone, succinonitrile, and dimethylvinylene carbonate can be exemplified. In addition, it is also possible to solidify the electrolyte for non-aqueous electrolyte batteries by gelling agents or cross-linking polymers, such as when used in non-aqueous electrolyte batteries called lithium polymer batteries.

### Lithium secondary battery

Furthermore, in an exemplary embodiment, the present invention provides a lithium secondary battery including:
an electrode assembly including a positive electrode comprising at least one among lithium metal oxide represented by Formula 2 and Formula 3 below, a negative electrode, and a separator interposed between the positive electrode and the negative electrode; and
the electrolyte composition according to the present invention:

   [Formula 2] Liₓ[Ni_{y}Co_{z}Mn_{w}M¹ᵥ]O₂

   [Formula 3] LiM²ₚMn₍₂₋ₚ₎O₄
in Formula 2 and Formula 3,
M¹ is at least one element selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w, and v are respectively 1.0≤x≤1.30, 0.5≤y<1, 0<z≤0.3, 0<w≤0.3, 0≤v≤0.1, y+z+w+v=1,
M² is Ni, Co, or Fe, and
p is 0.05≤p≤0.6.

The lithium secondary battery according to the present invention includes a positive electrode including a positive electrode active material, a negative electrode including a negative electrode active material, a separator interposed between the positive electrode and the negative electrode, and the lithium salt-containing non-aqueous electrolyte composition of the present invention as described above.

Specifically, the positive electrode includes a positive electrode composite layer prepared by applying, drying, and pressing a positive electrode active material onto a positive electrode current collector, which may optionally include a conductor, a binder, and other additives as required.

Here, the positive electrode active material is a material capable of electrochemically reacting on the positive electrode current collector, and includes at least one of the lithium metal oxides represented by Formula 2 and Formula 3, which are capable of reversibly intercalating and deintercalating lithium ions.

The lithium metal oxides represented by Formula 2 and Formula 3 are materials containing a high content of nickel (Ni) and manganese (Mn), respectively, and have the advantage of being able to stably supply high capacity and/or high voltage electricity when used as a positive electrode active material. In addition, when activating a secondary battery, a charging potential of 4.0 V or more is required to form a film on the positive electrode and/or negative electrode surface, but unlike conventional positive electrode active materials such as iron phosphate, which have a charging potential of less than about 4.0 V, the lithium metal oxides have a high charging potential of about 4.0 V or more, so that a film can be easily formed on the electrode.

Here, the lithium metal oxides represented by Formula 2 include LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂ and the like, and the lithium metal oxides represented by Formula 3 may include LiNi_{0.7}Mn_{1.3}O₄, LiNi_{0.5}Mn_{1.5}O₄, LiNi_{0.3}Mn_{1.7}O₄, and the like, which can be used alone or in combination.

In addition, the positive electrode can include a positive electrode current collector, which has a high conductivity without causing chemical changes in the cell. For example, stainless steel, aluminum, nickel, titanium, calcined carbon, and the like can be used, and in the case of aluminum or stainless steel, those that are surface-treated with carbon, nickel, titanium, silver, and the like can also be used. Furthermore, the average thickness of the current collector may be suitably applied from 3 to 500 µm, taking into account the conductivity and total thickness of the positive electrode being manufactured.

In addition, the negative electrode, like the positive electrode, has a negative electrode composite layer prepared by applying, drying, and pressing a negative electrode active material on a negative electrode current collector, and may optionally include a conductor, a binder, and other additives as required.

The negative electrode active material may include a carbon material and a silicon material. Specifically, the carbon material refers to a material having carbon atoms as the main component, and such carbon material may include at least one type selected from the group consisting of natural graphite, artificial graphite, expanded graphite, graphitized carbon, carbon black, acetylene black, and ketjen black. In addition, the silicon material refers to a material having silicon atoms as its main component, and such silicon material may include silicon (Si), silicon carbide (SiC), silicon monoxide (SiO), or silicon dioxide (SiO₂), either alone or in combination. When the silicon (Si) containing materials, silicon monoxide (SiO) and silicon dioxide (SiO₂) are homogeneously mixed or compounded and included in the negative electrode composite layer, they may be denoted as silicon oxide (SiO_{q}, wherein 0.8≤q≤2.5).

Moreover, the silicon material be included from 1 to 20 wt% based on the total weight of the negative electrode active material, more specifically from 3 to 10 wt%, from 8 to 15 wt%, from 13 to 18 wt%, or from 2 to 8 wt%. The present invention can maximize the energy density of the battery by adjusting the content of the silicon material to the above content range.

In addition, the negative electrode current collector is not particularly limited as long as it has a high conductivity without causing chemical changes in the cell, for example, copper, stainless steel, nickel, titanium, calcined carbon, and the like can be used, and in the case of copper or stainless steel, those that are surface-treated with carbon, nickel, titanium, silver, and the like can be used. Furthermore, the average thickness of the negative electrode current collector may be suitably applied from 1 to 500 µm, taking into account the conductivity and total thickness of the negative electrode to be manufactured.

Meanwhile, the separator interposed between the positive electrode and the negative electrode of each unit cell is an insulating thin film having high ionic permeability and mechanical strength, and is not particularly limited to those conventionally used in the art, but specifically, those that include one or more polymers among chemically resistant and hydrophobic polypropylene; polyethylene; and polyethylene-propylene copolymers. The separator may be in the form of a porous polymeric material such as a sheet or nonwoven fabric including the polymer described above, and in some cases may be in the form of a composite separator in which organic or inorganic particles are coated on the porous polymeric material by an organic binder. Furthermore, the separator may have an average pore diameter of 0.01 to 10 µm and an average thickness of 5 to 300 µm.

Further, the secondary battery includes a non-aqueous electrolyte composition according to the present invention as described above as an electrolyte.

The compounds having the structure represented by Formula 1 can uniformly form an organic and/or inorganic film on the surface of the positive electrode and/or negative electrode upon activation of a secondary battery including them. This uniformly formed organic and/or inorganic film can inhibit the decomposition of the electrolyte and the generation of gas when the cell is exposed to high temperature, and can improve the OCV drop and capacity degradation of the cell generated at the positive electrode, thereby further improving the performance and high temperature safety of the cell.

### Examples

Hereinafter, the present invention will be described in more detail by means of embodiments and experimental examples.

However, the following embodiments and experimental examples are only illustrative of the invention, and the contents of the invention are not limited to the following Examples and Experimental Examples.

### Examples 1-5 and Comparative Examples 1-6: Preparation of electrolyte composition for lithium secondary battery

A non-aqueous electrolyte composition was prepared by dissolving LiPF₆ as a lithium salt in a solvent containing ethylene carbonate (EC) and ethyl methyl carbonate (EMC) in a 3:7 volume ratio to a concentration of 1 M, and dissolving and weighing the electrolyte additives based on the total weight of the electrolyte as shown in Table 1 below.

**[Table 1]**

| | Type of non-aqueous electrolyte additive | Content |
|---|---|---|
| Example 1 | 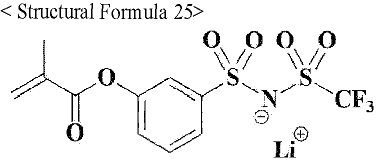 | 2 wt% |
| Example 2 | | 0.001 wt% |
| Example 3 | | 10 wt% |
| Example 4 | 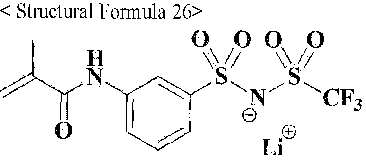 | 2 wt% |
| Example 5 | 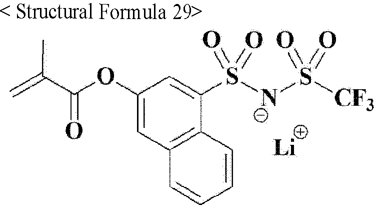 | 2 wt% |
| Example 6 | 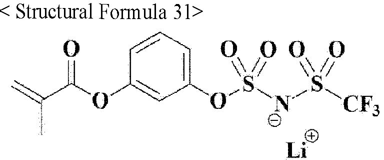 | 2 wt% |
| Example 7 | 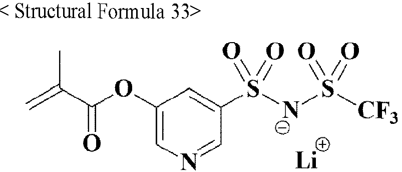 | 2 wt% |
| Comparative Example 1 | Not added | - |
| Comparative Example 2 | 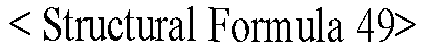 | 2 wt% |
| | 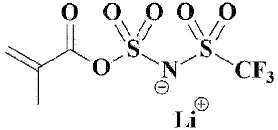 | |
| Comparative Example 3 | 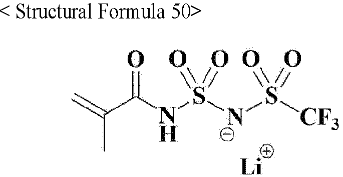 | 2 wt% |
| Comparative Example 4 | 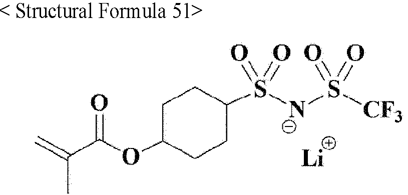 | 2 wt% |
| Comparative Example 5 | 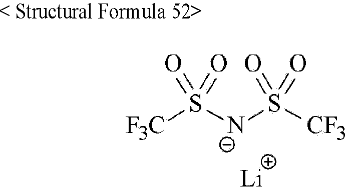 | 2 wt% |
| Comparative Example 6 | 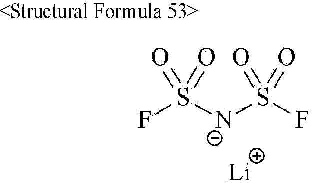 | 2 wt% |

### Comparative Example 7: Preparation of Electrolyte Composition for Lithium Secondary Battery

A non-aqueous electrolyte composition for a lithium secondary battery was prepared by performing the same method as in Example 1, except that an oligomer (weight average molecular weight: 2,500 to 5,000) polymerized with the compound represented by the following structural formula was used instead of the compound represented by Structural Formula 25 as an electrolyte additive.

### Examples 8-14 and Comparative Examples 8-14: Preparation of Lithium Secondary Battery

LiNi_{0.5}Mn_{1.5}O₄ with a particle size of 5 µm was prepared as a positive electrode active material, mixed with polyvinylidene fluoride as a carbon-based conductor and binder in a weight ratio of 94:3:3 in N-methylpyrrolidone (NMP) to form a slurry, cast on an aluminum thin plate, dried in a 120°C vacuum oven, and rolled to prepare a positive electrode.

Separately, a negative electrode active material composed of artificial graphite and silicon oxide (SiO₂) in a weight ratio of 9:1 was prepared, and 97 parts by weight of the negative electrode active material and 3 parts by weight of styrene butadiene rubber (SBR) were mixed with water to form a slurry, cast on a copper sheet, dried in a 130°C vacuum oven, and rolled to prepare a negative electrode.

A lithium secondary battery was prepared by interposing a separator made of 18 µm polypropylene on the above-obtained the positive electrode and negative electrode, inserting it into a case, and injecting the electrolyte composition prepared in Examples 1-7 and Comparative Examples 1-7 as shown in Table 2 below.

**[Table 2]**

| | Type of electrolyte composition |
|---|---|
| Example 8 | Electrolyte composition of Example 1 |
| Example 9 | Electrolyte composition of Example 2 |
| Example 10 | Electrolyte composition of Example 3 |
| Example 11 | Electrolyte composition of Example 4 |
| Example 12 | Electrolyte composition of Example 5 |
| Example 13 | Electrolyte composition of Example 6 |
| Example 14 | Electrolyte composition of Example 7 |
| Comparative Example 8 | Electrolyte composition of Comparative Example 1 |
| Comparative Example 9 | Electrolyte composition of Comparative Example 2 |
| Comparative Example 10 | Electrolyte composition of Comparative Example 3 |
| Comparative Example 11 | Electrolyte composition of Comparative Example 4 |
| Comparative Example 12 | Electrolyte composition of Comparative Example 5 |
| Comparative Example 13 | Electrolyte composition of Comparative Example 6 |
| Comparative Example 14 | Electrolyte composition of Comparative Example 7 |

### Experimental Example 1

In order to analyze the form in which the electrolyte composition for the lithium secondary battery according to the present invention exists in the secondary battery, the following experiments were performed on the electrolyte compositions prepared in Example 1 and Comparative Examples 1 and 7, respectively.

### A) Raman spectroscopic analysis

Raman spectroscopic analysis in the wavelength range of 650 to 760 cm⁻¹ was performed on the electrolyte composition (5 ml) prepared in Example 1 using a laser at 532 nm.

As a result, it was confirmed that the electrolyte composition of the embodiment comprising the electrolyte additive represented by Formula 1 according to the present invention has an increased band intensity in the Raman spectrum near 743±1 cm⁻¹ compared to the electrolyte composition prepared in Comparative Example 1 that does not contain the electrolyte additive. This is a phenomenon indicating that the negatively charged nitrogen atoms and positively charged lithium ions (Li⁺) contained in the sulfonylimide of the electrolyte additive represented by Formula 1 form coordination bonds between ionic substances, indicating that the electrolyte composition of Example 1 contains an ionic compound. In addition, the increase in band intensity as described above indicates that the ionic compound may undergo a reduction reaction on the negative electrode surface.

### B) Analysis of the differential capacity curve of the half cell

A half cell was prepared using lithium metal and graphite (artificial graphite: natural graphite=9:1 weight ratio mix), and the upper half cell was injected with the electrolyte compositions prepared in Examples 1 and 7, respectively. Then, the cells were charged from 3.5±0.5 V at 25°C to 0.05 V at a rate of 0.005C, the potential value (V) and the capacity value (mAh) were measured, and the reduction potential value was determined by differentiating the potential value with the capacity value (dQ/dV).

As a result, unlike the electrolyte compositions of Comparative Examples 1 and 7 that do not contain electrolyte additives or contain electrolyte additives in the form of oligomers, it was confirmed that the electrolyte composition of the Examples including the electrolyte additive represented by Formula 1 according to the present invention exhibits a falling peak at a voltage around 1.40 V relative to lithium. This falling peak indicates that a reduction reaction has occurred on the surface of the graphite electrode, which is the negative electrode, indicating that the electrolyte additive represented by Formula 1 included in the electrolyte composition is converted to the film material via a reduction reaction on the surface of the negative electrode at around 1.40 V relative to lithium.

### C) Evaluation of Linear Sweep Voltammetry of a tri-electrode cell

A tri-electrode cell was prepared by injecting the electrolyte compositions prepared in Example 1 and Comparative Examples 1 and 7 into a cell containing a platinum electrode, a platinum electrode, and a lithium metal electrode as three electrodes, respectively, and a Linear Sweep Voltammetry (LSV) analysis was performed on each of the fabricated cells. The linear sweep voltammetry was performed under the conditions of an observation range of 3.0 to 6.0 V (based on lithium), a step voltage of 50 mV and a measurement speed of 50 mV/s.

As a result, it can be seen that an electrolyte composition comprising an electrolyte additive represented by Formula 1 according to the present invention shows an increase in current around 4.0±0.05 V relative to lithium. This means that an oxidation reaction occurs on the surface of the lithium metal around 4.0±0.05 V, indicating that the electrolyte additive included in the electrolyte composition of Example 1 forms a film through an oxidation reaction on the surface of the positive electrode when a condition of 4.0±0.05 V relative to lithium is reached. Furthermore, it indicates that at the carbon electrode or positive electrode, the oxidation reaction is induced at a lower potential than at the platinum electrode surface due to the catalytic properties of the carbon or transition metal. On the other hand, the electrolyte compositions of Comparative Examples 1 and 7, which do not contain electrolyte additives or contain electrolyte additives in the form of oligomers, do not show an increase in current around 4.0±0.05V.

From these results, it can be seen that the electrolyte additive according to the present invention is an ionic material that performs oxidation and reduction reactions at the positive and negative electrodes of the battery during charging and discharging, respectively, and forms a film on the surface of each electrode.

### Experimental Example 2

The following experiments were performed to analyze the film formed on the surface of the electrode upon activation of the lithium secondary battery according to the present invention and to evaluate the high temperature safety of the lithium secondary battery. Here, the lithium secondary batteries tested were the secondary batteries of Examples 15-21 and Comparative Examples 15-21, which were prepared by performing the same method as Examples 8-14 and Comparative Examples 8-14, except that the lithium secondary batteries includedLiNi_{0.6}Co_{0.2}Mn_{0.2}O₂ as the positive electrode active material and artificial graphite as the negative electrode active material.

### A) Electrode surface film analysis

For the secondary cells of Example 15, Comparative Example 15, and Comparative Example 21, charge and discharge with a charge termination voltage of 4.2 V (NMC/graphite) and a discharge termination voltage of 2.5 V (NMC/graphite) at a charge/discharge current density of 0.33 C/0.33 C were performed three times each, and X-ray photoelectron spectroscopic (XPS) analysis was performed on the surface of the positive electrode and negative electrode of each cell in a fully discharged state.

As a result, the secondary battery (Example 15) including the electrolyte composition of Example 1 was found to have peaks in the range of 280 to 300 eV, representing the binding energy of carbon and fluorine, for both the positive electrode and negative electrode, during X-ray photoelectron spectroscopy (XPS) analysis of the electrode surface. Specifically, the positive electrode and negative electrode of the secondary battery exhibited a peak at 293±0.2 eV representing the binding energy of the CF₃ group, which means that the electrolyte additive represented by Formula 1 contained in the electrolyte composition participates in the formation of the film formed on the surface of the positive electrode and negative electrode. On the other hand, the secondary battery (Comparative Example 15) using the electrolyte composition of Comparative Example 1, which does not contain the electrolyte additive, and the secondary battery (Comparative Example 21) using the electrolyte composition of Comparative Example 7, which contains the electrolyte additive in oligomeric form, were found not to exhibit binding energy peaks originating from CF₃ groups at both the positive electrode and negative electrode.

From these results, it can be concluded that the electrolyte additive according to the present invention can form a film on the electrode surface by including an ionic compound in the form of a monomer represented by Formula 1 upon activation of the secondary battery.

### B) Evaluation of high temperature storage stability of the secondary battery

Each secondary battery was stored at 60°C for 56 days to observe ① the amount of gas generation in the secondary battery, ② the OCV of the secondary battery hourly, and ③ the capacity retention rate after high temperature storage.

Specifically, each secondary battery was charged and discharged three times with a charge/discharge current density of 0.33C/0.33C, a charge termination voltage of 4.2 V (NMC/graphite) and a discharge termination voltage of 2.5 V (NMC/graphite) to measure the battery capacity, and then charged and discharged in CC/CV mode with a charge termination voltage of 4.2 V at 0.33C to begin high temperature storage. At this time, the high temperature storage was stored in a 60°C constant temperature chamber for a total of 56 days. After 56 days, the pre- and post-storage OCV deviation (i.e., the degree of OCV drop) and capacity retention rate were measured, and the volume of gas generated by the secondary battery after high temperature storage was measured using the Archimedes principle, and the results are shown in Table 3 below.

**[Table 3]**

| | Type of electrolyte additive used | OCV deviation [mV] | Capacity retention rate [%] | Gas generated [ µℓ] |
|---|---|---|---|---|
| Example 15 | 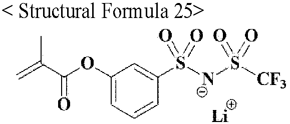 | 32.9 | 98.0 | 1313 |
| Example 16 | | 35.7 | 96.5 | 1826 |
| Example 17 | | 36.3 | 96.8 | 1971 |
| Example 18 | 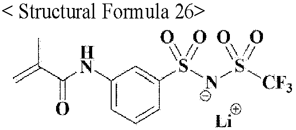 | 33.2 | 97.9 | 1325 |
| Example 19 | 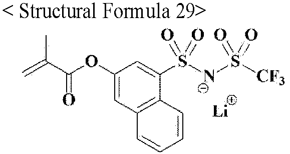 | 33.5 | 97.9 | 1361 |
| Example 20 | 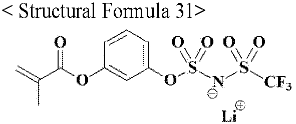 | 34.0 | 97.3 | 1407 |
| Example 21 | 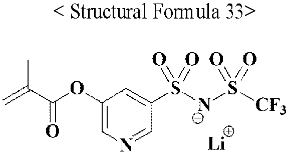 | 32.8 | 97.5 | 1388 |
| Comparative Example 15 | Not added | 38.5 | 95.6 | 2023 |
| | | | | |
| Comparative Example 16 | 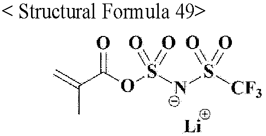 | 40.0 | 95.0 | 1710 |
| Comparative Example 17 | 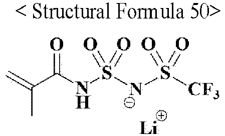 | 39.6 | 95.4 | 1943 |
| Comparative Example 18 | 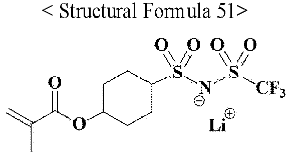 | 35.6 | 96.9 | 1560 |
| Comparative Example 19 | 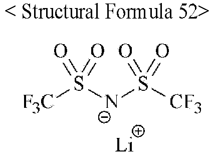 | 38.1 | 95.8 | 1943 |
| Comparative Example 20 | 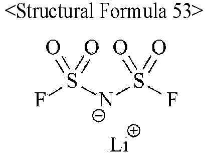 | 39.6 | 96.1 | 1928 |
| Comparative Example 21 | Olygomer derived from<Structural Formula 25> | 39.2 | 95.7 | 1877 |

As shown in Table 3 above, it can be seen that the secondary battery of the Examples including the compound represented by Formula 1 according to the present invention as an electrolyte additive significantly reduces the amount of gas generated due to the reduced decomposition of the electrolyte by the film formed on the surface of the positive electrode and negative electrode even when exposed to high temperature conditions, and reduces the phenomenon of OCV decline occurring at the positive electrode.

Although the above has been described with reference to a preferred embodiment of the present invention, it will be understood by a person skilled in the art or having ordinary knowledge in the art that various modifications and changes can be made to the present invention without departing from the field of thought and technology described in the following claims.

Therefore, the technical scope of the invention is not limited to what is described in the detailed description of the specification, but should be determined by the claims.

## Claims

1. An electrolyte composition for a secondary battery comprising a non-aqueous organic solvent, a lithium salt, and a compound represented by Formula 1 below: 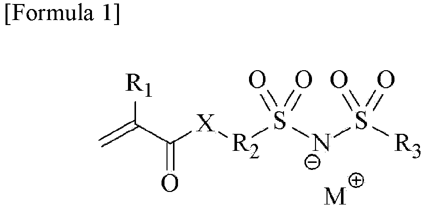 wherein
R₁ is hydrogen or an alkyl group having a carbon number of 1 to 4;
R₂ is an arylene group having from 6 to 20 carbons, an aryleneoxy group having from 6 to 20 carbons, a heteroarylene group having from 5 to 10 carbons and containing at least one heteroatom among N, S, and O, or a heteroaryleneoxy group having from 5 to 10 carbons and containing at least one heteroatom among N, S, and O;
R₃ is a fluoro group, an alkyl group having a carbon number of 1 to 10, an alkoxy group having a carbon number of 1 to 10, or 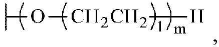 wherein at least one hydrogen in the alkyl group having a carbon number of 1 to 10, alkoxy group having a carbon number of 1 to 10, and 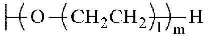 is optionally substituted by a fluorine atom;
X is an oxygen atom (O) or -NR₄;
R₄ is hydrogen or an alkyl group having a carbon number of 1 to 4;
M is at least one of lithium, sodium, and potassium;
l is an integer from 1 to 6; and
m is an integer from 2 to 20.

2. The electrolyte composition of claim 1, wherein, in Formula 1,
R₁ is hydrogen or a methyl group;
R₂ is a phenylene group, a naphthylene group, an anthracenyl group, a biphenylene group, a phenyleneoxy group, a pyridinylene group, a thiophenylene group, a dioxolene group, or a dithiolene group;
R₃ is a fluoro group, a methyl group, an ethyl group, a propyl group, a methoxy group, an ethoxy group, 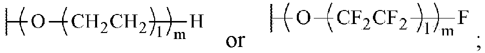
X is an oxygen atom (O), -NH, or -NCH₃;
M is lithium;
l is an integer of 1 or 2; and
m is an integer from 2 to 10.

3. The electrolyte composition of claim 1, wherein the compound represented by Formula 1 is one or more compounds of Structural Formula 1 to Structural Formula 48 below:
| | | |
|---|---|---|
| Structural Formula 1 | Structural Formula 2 | Structural Formula 3 |
| 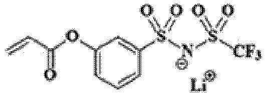 | 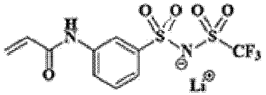 | 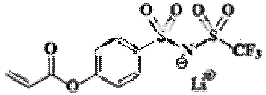 |
| Structural Formula 4 | Structural Formula 5 | Structural Formula 6 |
| 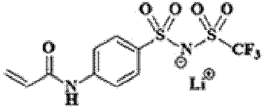 | 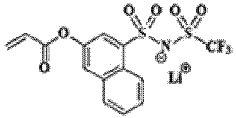 | 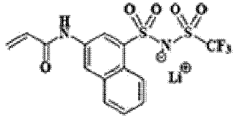 |
| Structural Formula 7 | Structural Formula 8 | Structural Formula 9 |
| 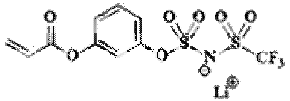 | 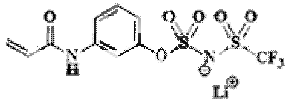 | 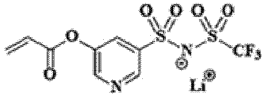 |
| Structural Formula 10 | Structural Formula 11 | Structural Formula 12 |
| 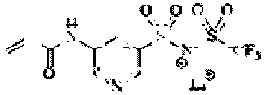 | 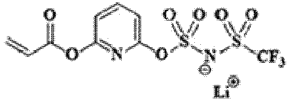 | 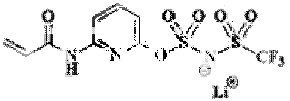 |
| Structural Formula 13 | Structural Formula 14 | Structural Formula 15 |
| 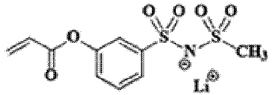 | 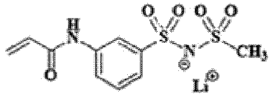 | 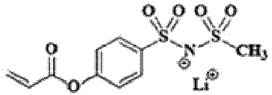 |
| Structural Formula 16 | Structural Formula 17 | Structural Formula 18 |
| 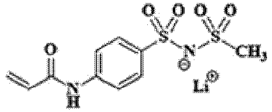 | 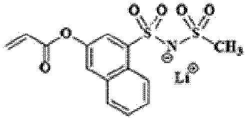 | 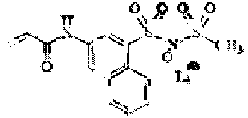 |
| Structural Formula 19 | Structural Formula 20 | Structural Formula 21 |
| 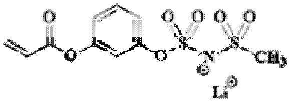 | 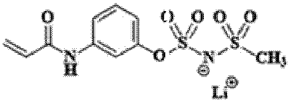 | 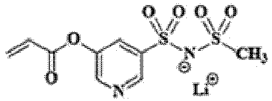 |
| Structural Formula 22 | Structural Formula 23 | Structural Formula 24 |
| 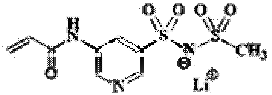 | 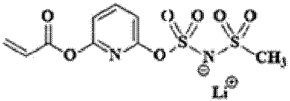 | 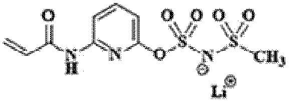 |
| Structural Formula 25 | Structural Formula 26 | Structural Formula 27 |
| 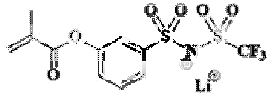 | 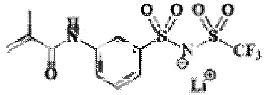 | 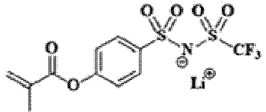 |
| Structural Formula 28 | Structural Formula 29 | Structural Formula 30 |
| 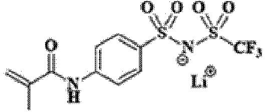 | 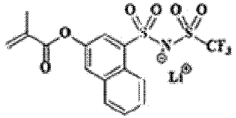 | 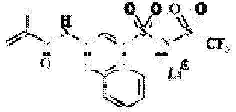 |
| Structural Formula 31 | Structural Formula 32 | Structural Formula 33 |
| 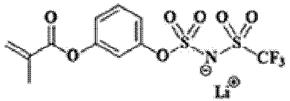 | 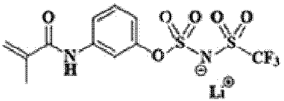 | 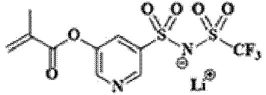 |
| Structural Formula 34 | Structural Formula 35 | Structural Formula 36 |
| 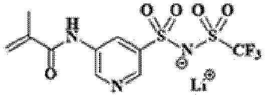 | 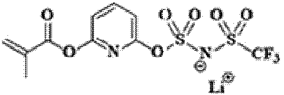 | 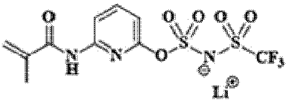 |
| | | |
|---|---|---|
| Structural Formula 37 | Structural Formula 38 | Structural Formula 39 |
| 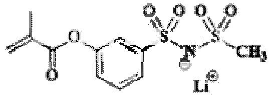 | 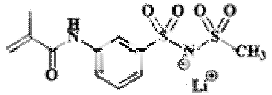 | 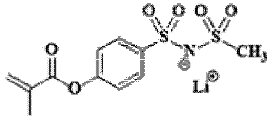 |
| Structural Formula 40 | Structural Formula 41 | Structural Formula 42 |
| 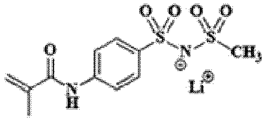 | 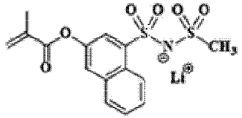 | 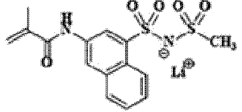 |
| Structural Formula 43 | Structural Formula 44 | Structural Formula 45 |
| 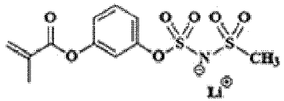 | 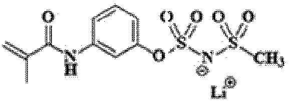 | 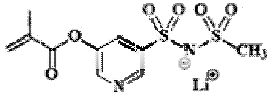 |
| Structural Formula 46 | Structural Formula 47 | Structural Formula 48 |
| 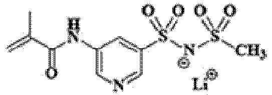 | 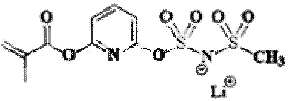 | 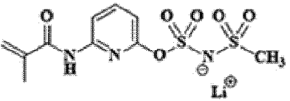 |

4. The electrolyte composition of claim 1, wherein the compound represented by Formula 1 is included in an amount of 0.01 to 3 wt% based on the total weight of the electrolyte composition.

5. The electrolyte composition of claim 1, wherein the lithium salt includes at least one of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

6. The electrolyte composition of claim 1, wherein the non-aqueous organic solvent includes at least one of N-methyl-2-pyrrolidinone, ethylene carbonate, propylene carbonate, butylene carbonate, dimethyl carbonate, diethyl carbonate, gamma-butyrolactone, 1,2-dimethoxyethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl acetate, phosphoric acid triester, trimethoxymethane, dioxolane derivative, sulfurane, methyl sulfurane, 1,3-dimethyl-2-imidazolidinone, propylene carbonate derivative, tetrahydrofuran derivative, ether, methyl pyrophosphate, and ethyl propionate.

7. A lithium secondary battery comprising:
an electrode assembly comprising a positive electrode comprising at least one lithium metal oxide selected from compounds represented by Formula 2 and Formula 3 below, a negative electrode comprising a negative electrode active material, and a separator interposed between the positive electrode and the negative electrode; and
the electrolyte composition according to claim 1;
[Formula 2] Liₓ[Ni_{y}Co_{z}Mn_{w}M¹ᵥ]O₂
[Formula 3] LiM²ₚMn₍₂₋ₚ₎O₄
wherein, in Formula 2 and Formula 3,
M¹ is at least one of W, Cu, Fe, V, Cr, Ti, Zr, Zn, Al, In, Ta, Y, La, Sr, Ga, Sc, Gd, Sm, Ca, Ce, Nb, Mg, B, and Mo;
1.0≤x≤1.30, 0.5≤y<1, 0<z≤0.3, 0<w≤0.3, 0≤v≤0.1, and y+z+w+v=1;
M² is Ni, Co, or Fe; and
p is 0.05≤p≤0.6.

8. The lithium secondary battery of claim 7, wherein the positive electrode active material comprises at least one of LiNi_{0.3}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂, and LiNi_{0.5}Mn_{1.5}O_{4.}

9. The lithium secondary battery of claim 7, wherein the negative electrode active material comprises a carbon material and a silicon material, wherein the silicon material includes at least one of silicon (Si), silicon carbide (SiC), and silicon oxide SiO_{q}, wherein 0.8≤q≤2.5.

10. The lithium secondary battery of claim 9, wherein the silicon material is included at 1 to 20 wt% based on the total weight of the negative electrode active material.

## Patentansprüche

1. Elektrolytzusammensetzung für eine Sekundärbatterie, umfassend ein nichtwässriges organisches Lösungsmittel, ein Lithiumsalz und eine durch die nachstehende Formel 1 dargestellte Verbindung: 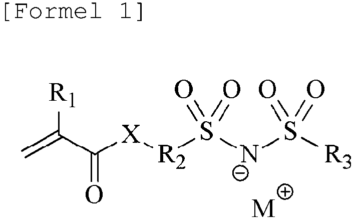 worin
R₁ Wasserstoff oder eine Alkylgruppe mit einer Kohlenstoffanzahl von 1 bis 4 ist,
R₂ Arylengruppe mit 6 bis 20 Kohlenstoffen, eine Arylenoxygruppe mit 6 bis 20 Kohlenstoffen, eine Heteroarylengruppe, die 5 bis 10 Kohlenstoffen aufweist und mindestens ein Heteroatom unter N, S und O enthält, oder eine Heteroarylenoxygruppe, die 5 bis 10 Kohlenstoffe aufweist und mindestens ein Heteroatom unter N, S und O aufweist, ist,
R₃ eine Fluorgruppe, eine Alkylgruppe mit einer Kohlenstoffanzahl von 1 bis 10, eine Alkoxygruppe mit einer Kohlenstoffanzahl von 1 bis 10 oder 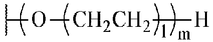 ist, wobei mindestens ein Wasserstoff in der Alkylgruppe mit einer Kohlenstoffanzahl von 1 bis 10, Alkoxygruppe mit einer Kohlenstoffanzahl von 1 bis 10 und 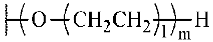 optional durch ein Fluoratom substituiert ist,
X ein Sauerstoffatom (O) oder -NR₄ ist,
R₄ Wasserstoff oder eine Alkylgruppe mit einer Kohlenstoffanzahl von 1 bis 4 ist,
M mindestens eines von Lithium, Natrium und Kalium ist,
l eine ganze Zahl von 1 bis 6 ist und
m eine ganze Zahl von 2 bis 20 ist.

2. Elektrolytzusammensetzung nach Anspruch 1, wobei in Formel 1
R₁ Wasserstoff oder eine Methylgruppe ist,
R₂ eine Phenylengruppe, eine Naphthylengruppe, eine Anthracenylgruppe, eine Biphenylengruppe, eine Phenlyenoxygruppe, eine Pyridinylengruppe, eine Thiophenylengruppe, eine Dioxolengruppe oder eine Dithiolengruppe ist,
R₃ eine Fluorgruppe, eine Methylgruppe, eine Ethylgruppe, eine Propylgruppe, eine Methoxygruppe, eine Ethoxygruppe, 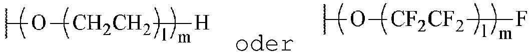 ist,
X ein Sauerstoffatom (0), -NH oder -NCH₃ ist,
M Lithium ist,
l eine ganze Zahl von 1 oder 2 ist und
m eine ganze Zahl von 2 bis 10 ist.

3. Elektrolytzusammensetzung nach Anspruch 1, wobei die durch die Formel 1 dargestellte Verbindung eine oder mehrere Verbindungen der nachstehenden Strukturformel 1 bis Strukturformel 48 ist:
| | | |
|---|---|---|
| Strukturformel 1 | Strukturformel 2 | Strukturformel 3 |
| 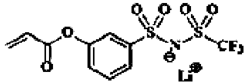 | 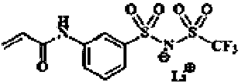 | 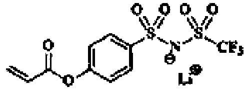 |
| Strukturformel 4 | Strukturformel 5 | Strukturformel 6 |
| 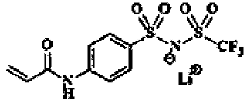 | 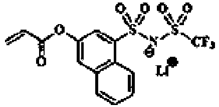 | 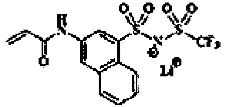 |
| Strukturformel 7 | Strukturformel 8 | Strukturformel 9 |
| 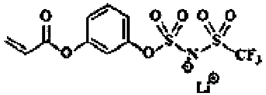 | 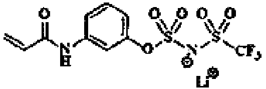 | 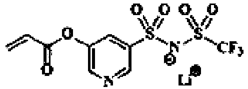 |
| Strukturformel 10 | Strukturformel 11 | Strukturformel 12 |
| 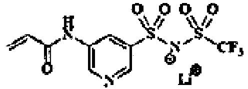 | 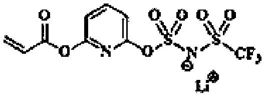 | 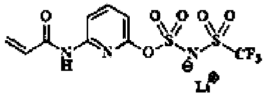 |
| Strukturformel 13 | Strukturformel 14 | Strukturformel 15 |
| 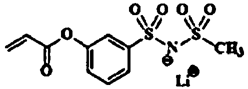 | 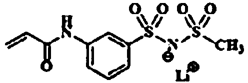 | 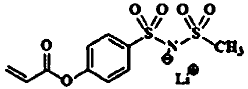 |
| Strukturformel 16 | Strukturformel 17 | Strukturformel 18 |
| 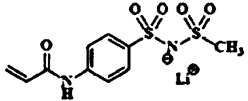 | 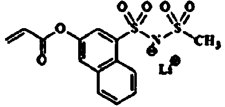 | 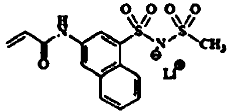 |
| Strukturformel 19 | Strukturformel 20 | Strukturformel 21 |
| 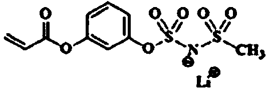 | 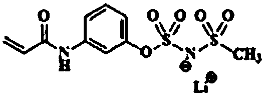 | 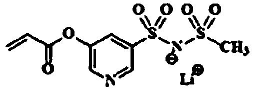 |
| Strukturformel 22 | Strukturformel 23 | Strukturformel 24 |
| 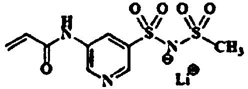 | 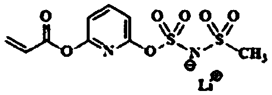 | 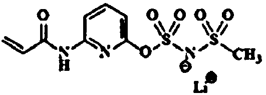 |
| | | |
|---|---|---|
| Strukturformel 25 | Strukturformel 26 | Strukturformel 27 |
| 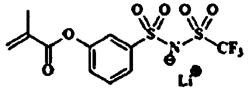 | 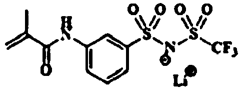 | 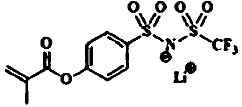 |
| **Strukturformel** 28 | Strukturformel 29 | Strukturformel 30 |
| 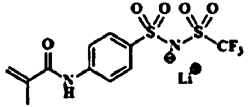 | 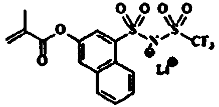 | 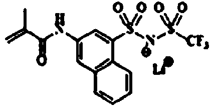 |
| Strukturformel 31 | Strukturformel 32 | **Strukturformel** 33 |
| 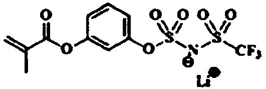 | 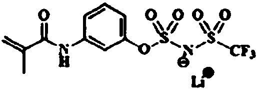 | 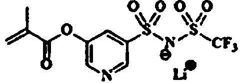 |
| Strukturformel 34 | Strukturformel 35 | Strukturformel 36 |
| 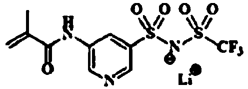 | 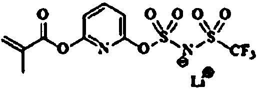 | 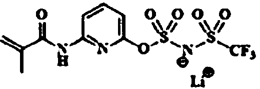 |
| Strukturformel 37 | Strukturformel 38 | Strukturformel 39 |
| 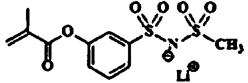 | 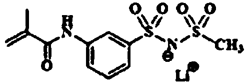 | 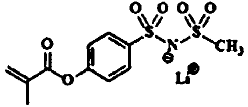 |
| Strukturformel 40 | Strukturformel 41 | Strukturformel 42 |
| 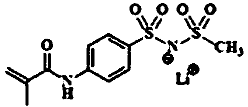 | 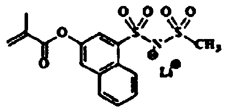 | 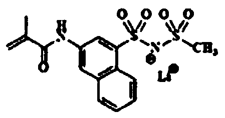 |
| Strukturformel 43 | Strukturformel 44 | Strukturformel 45 |
| 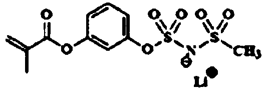 | 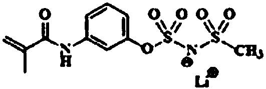 | 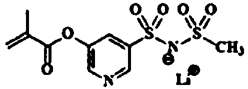 |
| Strukturformel 46 | Strukturformel 47 | Strukturformel 48 |
| 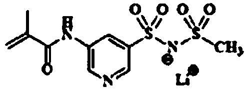 | 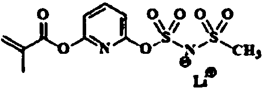 | 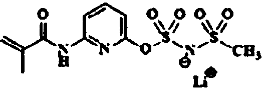 |

4. Elektrolytzusammensetzung nach Anspruch 1, wobei die durch Formel 1 dargestellte Verbindung in einer Menge von 0,01 bis 3 Gew.-%, bezogen auf das Gesamtgewicht der Elektrolytzusammensetzung, enthalten ist.

5. Elektrolytzusammensetzung nach Anspruch 1, wobei das Lithiumsalz mindestens eines von LiCl, LiBr, LiI, LiClO₄, LiBF₄, LiB₁₀Cl₁₀, LiPF₆, LiCF₃SO₃, LiCF₃CO₂, LiAsF₃, LiSbF₆, LiAlCl₄, CH₃SO₃Li, (CF₃SO₂)₂NLi und (FSO₂)₂NLi einschließt.

6. Elektrolytzusammensetzung nach Anspruch 1, wobei das nichtwässrige organische Lösungsmittel mindestens eines von N-Methyl-2-pyrrolidinon, Ethylencarbonat, Propylencarbonat, Butylencarbonat, Dimethylcarbonat, Diethylcarbonat, gamma-Butyrolacton, 1,2-Dimethoxyethan, Tetrahydrofuran, 2-Methyltetrahydrofuran, Dimethylsulfoxid, 1,3-Dioxolan, Formamid, Dimethylformamid, Dioxolan, Acetonitril, Nitromethan, Methylformiat, Methylacetat, Phosphorsäuretriester, Trimethoxymethan, Dioxolanderivat, Sulfuran, Methylsulfuran, 1,3-Dimethyl-2-imidazolidinon, Propylencarbonatderivat, Tetrahydrofuranderivat, Ether, Methylpyrophosphat und Ethylpropion einschließt.

7. Lithiumsekundärbatterie, umfassend:
eine Elektronenanordnung, umfassend eine positive Elektrode, die mindestens ein Lithiummetalloxid, ausgewählt aus durch die nachstehende Formel 2 und Formel 3 dargestellten Verbindungen, umfasst, eine negative Elektrode, die ein negatives Elektrodenaktivmaterial umfasst, und einen Separator, der zwischen der positiven Elektrode und der negativen Elektrode eingefügt ist, und
die Elektrolytzusammensetzung gemäß Anspruch 1,
[Formel 2] Liₓ[Ni_{y}CO_{z}Mn_{w}M¹_{y}]O₂
[Formel 3] LiM²ₚMn₂₋ₚO4
wobei in Formel 2 und Formel 3
M¹ mindestens eines von W, Cu, Fe, V, Cr, Ti, Zr, Zn, Al, In, Ta, Y, La, Sr, Ga, Sc, Gd, Sm, Ca, Ce, Nb, Mg, B und Mo ist,
1,0≤x≤1,30, 0,5≤y<1, 0<z≤0,3, 0<w≤0,3, 0≤v≤0,1 und y+z+w+v=1,
M² Ni, Co oder Fe ist und
p 0,05≤p≤0,6 ist.

8. Lithiumsekundärbatterie nach Anspruch 7, wobei das positive Elektrodenaktivmaterial mindestens eines von LiNi_{0,8}Co_{0,1}Mn_{0,1}O₂, LiNi_{0,6}Co_{0,2}Mn_{0,2}O₂, LiNi_{0,9}Co_{0,05}Mn_{0,05}O₂, LiNi_{0,6}Co_{0,}2Mn_{0,1}Al_{0,1}O₂, LiNi_{0,6}Co_{0,2}Mn_{0,15}Al_{0,05}O₂, LiNi_{0,7}Co_{0,1}Mn_{0,1}Al_{0,1}O₂ und LiNi_{0,5}Mn_{1,5}O₄ umfasst.

9. Lithiumsekundärbatterie nach Anspruch 7, wobei das negative Elektrodenaktivmaterial ein Kohlenstoffmaterial und ein Siliciummaterial umfasst, wobei das Siliciummaterial mindestens eines von Silicium (Si), Siliciumcarbid (SiC) und Siliciumoxid SiO_{q}, worin 0,8≤q≤2,5, einschließt.

10. Lithiumsekundärbatterie nach Anspruch 9, wobei das Siliciummaterial zu 1 bis 20 Gew.-%, bezogen auf das Gesamtgewicht des negativen Elektrodenaktivmaterials enthalten ist.

## Revendications

1. Composition d'électrolyte pour une batterie secondaire comprenant un solvant organique non aqueux, un sel de lithium et un composé représenté par la Formule 1 ci-dessous : 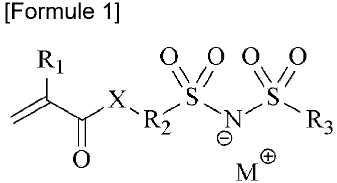 dans laquelle
R₁ est de l'hydrogène ou un groupe alkyle présentant un nombre d'atomes de carbone de 1 à 4 ;
R₂ est un groupe arylène présentant de 6 à 20 atomes de carbone, un groupe arylénoxy présentant de 6 à 20 atomes de carbone, un groupe hétéroarylène présentant de 5 à 10 atomes de carbone et contenant au moins un hétéroatome parmi N, S et O, ou un groupe hétéroarylénoxy présentant de 5 à 10 atomes de carbone et contenant au moins un hétéroatome parmi N, S et O ;
R₃ est un groupe fluoro, un groupe alkyle présentant de 1 à 10 atomes de carbone, un groupe alcoxy présentant de 1 à 10 atomes de carbone, ou 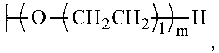 dans lequel au moins un hydrogène dans le groupe alkyle présentant de 1 à 10 atomes de carbone, le groupe alcoxy présentant de 1 à 10 atomes de carbone, et 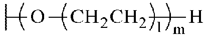 est facultativement substitué par un atome de fluor ;
X est un atome d'oxygène (O) ou -NR₄ ;
R₄ est de l'hydrogène ou un groupe alkyle présentant un nombre d'atomes de carbone de 1 à 4 ;
M est au moins un élément parmi le lithium, le sodium et le potassium ;
l est un nombre entier de 1 à 6 ; et
m est un nombre entier de 2 à 20.

2. Composition d'électrolyte selon la revendication 1, dans laquelle, dans la Formule 1,
R₁ est de l'hydrogène ou un groupe méthyle ;
R₂ est un groupe phénylène, un groupe naphtylène, un groupe anthracényle, un groupe biphénylène, un groupe phénylénoxy, un groupe pyridinylène, un groupe thiophénylène, un groupe dioxolène, ou un groupe dithiolène ;
R₃ est un groupe fluoro, un groupe méthyle, un groupe éthyle, un groupe propyle, un groupe méthoxy, un groupe éthoxy, 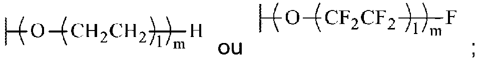
X est un atome d'oxygène (O), -NH, ou -NCH₃ ;
M est le lithium ;
l est un nombre entier égal à 1 ou 2 ; et
m est un nombre entier de 2 à 10.

3. Composition d'électrolyte selon la revendication 1, dans laquelle le composé représenté par la Formule 1 est un ou plusieurs composés des Formules structurelles 1 à 48 ci-dessous :
| | | |
|---|---|---|
| Formule structurelle 1 | Formule structurelle 2 | Formule structurelle 3 |
| 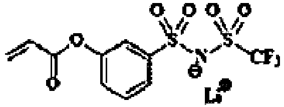 | 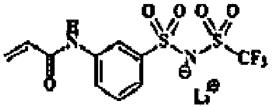 | 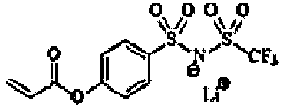 |
| Formule structurelle 4 | Formule structurelle 5 | Formule structurelle 6 |
| 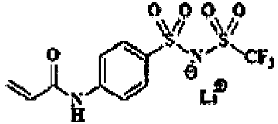 | 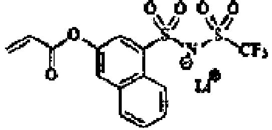 | 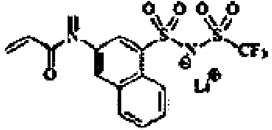 |
| Formule structurelle 7 | Formule structurelle 8 | Formule structurelle 9 |
| 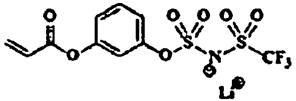 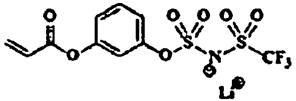 | 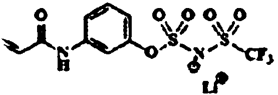 | 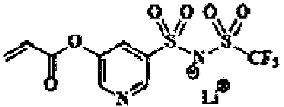 |
| Formule structurelle 10 | Formule structurelle 11 | Formule structurelle 12 |
| 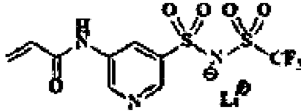 | 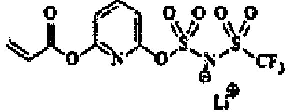 | 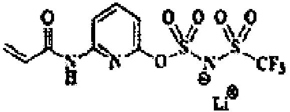 |
| Formule structurelle 13 | Formule structurelle 14 | Formule structurelle 15 |
| 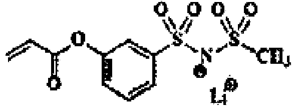 | 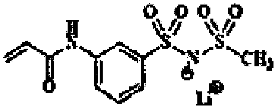 | 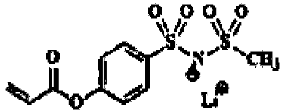 |
| Formule structurelle 16 | Formule structurelle 17 | Formule structurelle 18 |
| 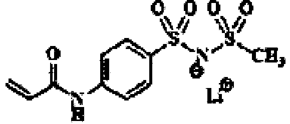 | 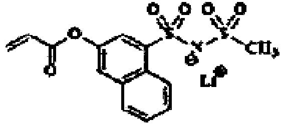 | 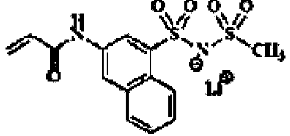 |
| Formule structurelle 19 | Formule structurelle 20 | Formule structurelle 21 |
| 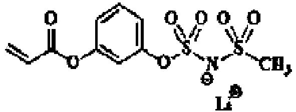 | 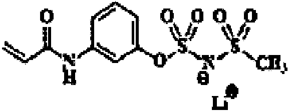 | 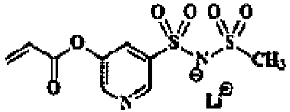 |
| Formule structurelle 22 | Formule structurelle 23 | Formule structurelle 24 |
| 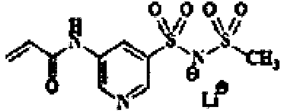 | 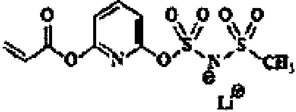 | 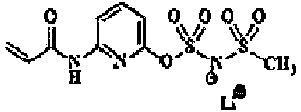 |
| Formule structurelle 25 | Formule structurelle 26 | Formule structurelle 27 |
| 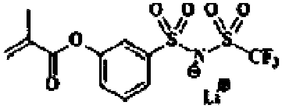 | 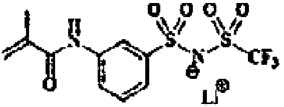 | 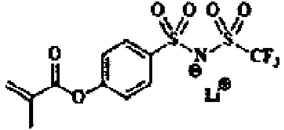 |
| Formule structurelle 28 | Formule structurelle 29 | Formule structurelle 30 |
| 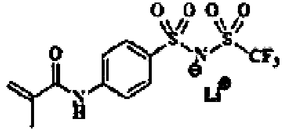 | 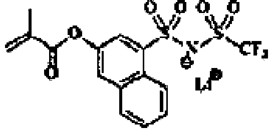 | 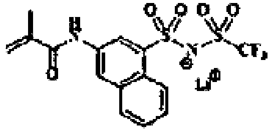 |
| Formule structurelle 31 | Formule structurelle 32 | Formule structurelle 33 |
| 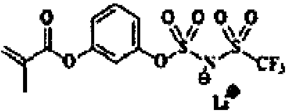 | 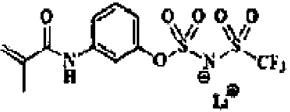 | 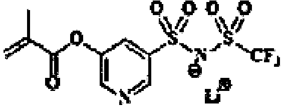 |
| Formule structurelle 34 | Formule structurelle 35 | Formule structurelle 36 |
| 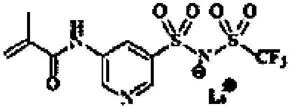 | 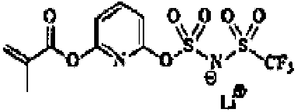 | 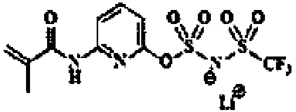 |
| Formule structurelle 37 | Formule structurelle 38 | Formule structurelle 39 |
| 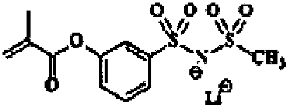 | 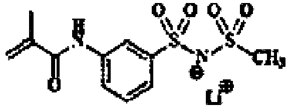 | 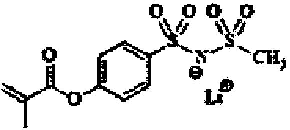 |
| Formule structurelle 40 | Formule structurelle 41 | Formule structurelle 42 |
| 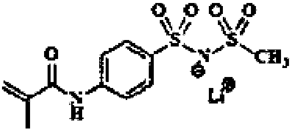 | 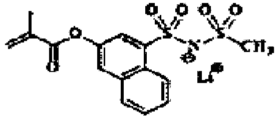 | 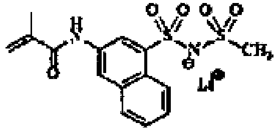 |
| Formule structurelle 43 | Formule structurelle 44 | Formule structurelle 45 |
| 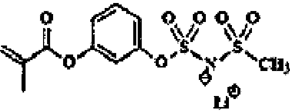 | 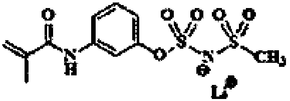 | 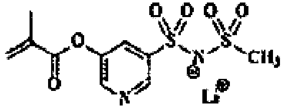 |
| Formule structurelle 46 | Formule structurelle 47 | Formule structurelle 48 |
| 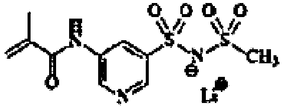 | 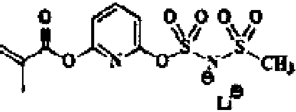 | 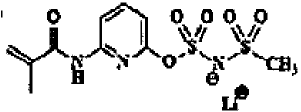 |

4. Composition d'électrolyte selon la revendication 1, dans laquelle le composé représenté par la Formule 1 est inclus en une quantité de 0,01 à 3 % en poids sur la base du poids total de la composition d'électrolyte.

5. Composition d'électrolyte selon la revendication 1, dans laquelle le sel de lithium inclut au moins un composé parmi LiCl, LiBr, Lil, LiCIO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, et (FSO₂)₂NLi.

6. Composition d'électrolyte selon la revendication 1, dans laquelle le solvant organique non aqueux comprend au moins un composé parmi la N-méthyl-2-pyrrolidinone, le carbonate d'éthylène, le carbonate de propylène, le carbonate de butylène, le carbonate de diméthyle, le carbonate de diéthyle, la γ-butyrolactone, 1,2-dim ethoxy ethane, le tétrahydrofurane, le 2-méthyl tétrahydrofurane, le diméthylsulfoxyde, le 1,3-dioxolane, la formamide, la diméthylformamide, le dioxolane, l'acétonitrile, le nitrométhane, le formiate de méthyle, l'acétate de méthyle, un triester de l'acide phosphorique, le triméthoxyméthane, un dérivé de dioxolane, le sulfurane, le méthyl sulfurane, la 1,3-diméthyl-2-imidazolidinone, un dérivé de carbonate de propylène, un dérivé de tétrahydrofurane, un éther, le pyrophosphate de méthyle et le propionate d'éthyle.

7. Batterie secondaire au lithium comprenant :
un ensemble d'électrodes comprenant une électrode positive comprenant au moins un oxyde de métal de lithium sélectionné parmi les composés représentés par la Formule 2 et la Formule 3 ci-dessous, une électrode négative comprenant un matériau actif d'électrode négative, et un séparateur interposé entre l'électrode positive et l'électrode négative ; et
la composition d'électrolyte selon la revendication 1 ;
[Formule 2] Liₓ[Ni_{y}Co_{z}Mn_{w}M¹ᵥ]O₂
[Formule 3] LiM²ₚMn(₂₋ₚ)O₄
dans laquelle, dans les Formules 2 et 3,
M¹ est au moins un élément parmi W, Cu, Fe, V, Cr, Ti, Zr, Zn, Al, In, Ta, Y, La, Sr, Ga, Sc, Gd, Sm, Ca, Ce, Nb, Mg, B et Mo ;
1,0 ≤ x ≤ 1,30, 0,5 ≤ y < 1, 0 < z ≤ 0,3, 0 < w ≤ 0,3, 0 ≤ v ≤ 0,1, et y+z+w+v = 1 ;
M² est Ni, Co ou Fe ; et
p est 0,05≤p≤0,6.

8. Batterie secondaire au lithium selon la revendication 7, dans laquelle le matériau actif d'électrode positive comprend au moins un composé parmi LiNi_{0,8}Co_{0,1}Mn_{0,1}O₂, LiNi_{0,6}Co_{0,2}Mn_{0,2}O₂, LiNi_{0,9}Co_{0,05}Mn_{0,05}O₂, LiNi_{0,6}Co_{0,2}Mn_{0,1}AL_{0,1}O₂, LiNi_{0,6}Co_{0,2}Mn_{0,15}Al_{0,05}O₂, LiNi_{0,7}Co_{0,1}Mn_{0,1}AL_{0,1}O₂, et LiNi_{0,5}Mn_{1,5}O₄.

9. Batterie secondaire au lithium selon la revendication 7, dans laquelle le matériau actif d'électrode négative comprend un matériau à base de carbone et un matériau à base de silicium, dans laquelle le matériau en silicium comprend au moins un élément parmi le silicium (Si), le carbure de silicium (SiC), et l'oxyde de silicium SiO_{q}, dans lequel 0,8 ≤ q ≤ 2,5.

10. Batterie secondaire au lithium selon la revendication 9, dans laquelle le matériau en silicium est inclus à raison de 1 à 20 % en poids sur la base du poids total du matériau actif d'électrode négative.
